(19) Europäisches Patentamt
European Patent Office
Office européen des brevets

(11) EP 0 644 925 B1

(12) EUROPEAN PATENT SPECIFICATION

(45) Date of publication and mention of the grant of the patent: **19.03.1997 Bulletin 1997/12**

(21) Application number: **93914271.7**

(22) Date of filing: **01.06.1993**

(51) Int Cl.[6]: **C11D 1/62**

(86) International application number: **PCT/US93/05158**

(87) International publication number: **WO 93/25648 (23.12.1993 Gazette 1993/30)**

# (54) STABLE BIODEGRADABLE FABRIC SOFTENING COMPOSITIONS

STABILE, BIOLOGISCH ABBAUBARE GEWEBEWEICHMACHERZUSAMMENSETZUNGEN

COMPOSITIONS ADOUCISSANTES, BIODEGRADABLES ET STABLES, POUR TISSUS

(84) Designated Contracting States:
**AT BE CH DE DK ES FR GB GR IE IT LI LU NL PT SE**

(30) Priority: **10.06.1992 US 896266**

(43) Date of publication of application: **29.03.1995 Bulletin 1995/13**

(73) Proprietor: **THE PROCTER & GAMBLE COMPANY Cincinnati, Ohio 45202 (US)**

(72) Inventors:
- **LICHTENWALTER, Glen, Dale Corsicana, TX 75110 (US)**
- **MILLER, Larry, Eugene Cincinnati, OH 45241 (US)**
- **SIRAM, Chiranjeevi Corsicana, TX 75110 (US)**
- **WAHL, Errol, Hoffman Cincinnati, OH 45242 (US)**

(74) Representative: **Canonici, Jean-Jacques et al
Procter & Gamble European Technical Center N.V.
Temselaan 100
1853 Strombeek-Bever (BE)**

(56) References cited:

| | |
|---|---|
| EP-A- 0 021 431 | EP-A- 0 239 910 |
| EP-A- 0 409 502 | EP-A- 0 507 478 |
| WO-A-91/17975 | DE-A- 1 903 955 |
| US-A- 4 370 272 | US-A- 5 066 414 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 644 925 B1

## Description

### TECHNICAL FIELD

The present invention relates to textile treatment compounds. In particular, it relates to biodegradable quaternary ammonium fabric softening compounds for use in textile treatment compositions that are used in the rinse cycle of a textile laundering operation to provide fabric softening/static control benefits. The compounds herein can also be used in hair conditioner compositions.

### BACKGROUND OF THE INVENTION

Textile treatment compositions suitable for providing fabric softening and static control benefits during laundering are well-known in the art and have found wide-scale commercial application. Historically, rinse-added fabric softening compositions contain, as the active softening component, substantially water-insoluble cationic materials having two long alkyl chains. Typical of such materials are ditallow dimethyl ammonium chloride and imidazolinium compounds substituted with two stearyl groups. These materials are normally prepared in the form of a dispersion in water.

It would be desirable to have fabric softening compounds which are storage-stable, and also which are biodegradable. However, materials which may be biodegradable are often difficult to formulate as stable liquid compositions.

The use of various quaternized ester-amines as cationic fabric softening agents is known in the art. See, for example, U.S. Pat. No. 4,339,391, Hoffmann, et al., issued July 13, 1982; U.S. Pat. No. 5,066,414, Chang, issued Nov. 19, 1991; U.S. Pat. No. 4,874,554, Lange et al., issued Oct. 17, 1989; U.S. Pat. No. 4,456,554, Walz et al., issued Jun. 26, 1984; U.S. Pat. No. 4,767,547, Straathof/Konig, issued Aug. 30, 1988; U.S. Pat. No. 3,915,867, Kang et al., issued Oct. 28, 1975; and U.S. Pat. No. 4,844,823, Jacques et al., issued July 4, 1989, EP 0,409,502 and WO 93/17085 (54.3 document), for a series of quaternized ester-amines which function as fabric softeners. Various quaternized ester-amines are commercially available under the trade names SYNPROLAM FS from ICI and REWOQUAT from REWO.

Quaternized ester-amines are believed to be rapidly biodegradable, probably because they are more subject to hydrolysis than are conventional cationic softening agents (e.g., ditallow dimethyl ammonium chloride and analogs thereof). As a result of hydrolysis, they can encounter hydrolytic stability problems upon prolonged shelf storage with the product stability and viscosity problems becoming increasingly more unmanageable in concentrated aqueous dispersions. However, improved biodegradability and additional benefits are still desirable.

Various solutions to the problem of preparing concentrated fabric softening compositions suitable for consumer use have been addressed in the art. See, for example, U.S. Pat. Nos. 4,426,299, issued Jan. 17, 1984, and 4,401,578, issued Aug. 30, 1983, Verbruggen, (paraffin, fatty acids and ester viscosity control agents); European Patent 0,018,039, Clint et al., issued March 7, 1984, (hydrocarbons plus soluble cationic or nonionic surfactants to improve viscosity and stability characteristics); U.S. Pat. No. 4,454,049, MacGilp et al., issued June 12, 1984, (isotropic solutions comprising water-insoluble di-$C_{16}$-$C_{24}$ optionally hydroxy-substituted alkyl, alkaryl or alkenyl cationic fabric softeners, at least 70% of the fabric softener consisting of one or more components together having a melting completion temperature of less than 20°C, a water-insoluble nonionic extender, especially $C_{10}$-$C_{40}$ hydrocarbons or esters of mono- or polyhydric alcohols with $C_8$-$C_{24}$ fatty acids, and a water-miscible organic solvent) U.S. Pat. No. 4,439,330, Ooms, issued March 27, 1984, (ethoxylated amines); and U.S. Pat. No. 4,476,031, Ooms, issued Oct. 9, 1984, (ethoxylated amines or protonated derivatives thereof, in combination with ammonium and imidazolinium materials). It is generally known (for example, in U.S. Pat. No. No. 3,681,241, Rudy, issued Aug. 1, 1972) that the presence of ionizable salts in softener compositions helps reduce viscosity.

EP 0,021,431 discloses a process for producing quaternary ammonium ester compounds having two long fatty alcohol groups.

DE 1,903,955 discloses alkali-stabilised emulsions comprising an ester of quaternary ammonium compounds with two hydrophobic chains. One hydrophobic chain is a fatty alkyl chain while the other is provided by a fatty alcohol group.

### SUMMARY OF THE INVENTION

The present invention relates to liquid fabric softening and antistatic compositions formulated at a pH of from 2.0 to 5.0 comprising

(a) from 1% to 40% of a quaternized ester amine softening compound having the formula :

$$\begin{array}{l} R_2\text{-}N^{\oplus}\text{-}[(CH_2)_n\text{-}C(O)\text{-}O\text{-}R^2]_m \qquad X^{\ominus} \\ \quad\ | \\ \quad R^1(2\text{-}m) \end{array}$$

wherein each R substituent is a short chain $C_1$-$C_6$ alkyl or hydroxyalkyl group, or mixtures thereof; $R^1$ is $(CH_2)_n$-O-(O)C-$R^2$ or $R^2$ group; $R^2$ is a $C_{13}$-$C_{22}$ hydrocarbyl group; m is 1 or 2; n is from 1 to 4; and $X^{\ominus}$ is a softener compatible anion;

(b) a stabilising agent selected from a linear ethoxylated alcohol in amount of from 0.1% to 2% by weight, a nonionic emulsifier in amount of from 0.1% to 2.5% by weight and mixtures thereof;

(c) from 60% to 99% of liquid carrier.

The ester groups in which the acyl group is attached to the nitrogen atom (reverse esters) are likely more hydrolytically unstable than the corresponding ester groups where the alcohol is attached to the nitrogen atom. This increased instability is believed to provide improved biodegradability. Such compounds, however, are more difficult to stabilize in aqueous compositions.

The reverse ester moieties lend biodegradability to these softening compounds. Since the fabric softening compounds used in these compositions are cationic, these compositions provide not only fiber and fabric softness, but also anti-static benefits.

The present invention encompasses liquid fabric softening and antistatic compositions, comprising from 1% to 40% by weight of a fabric softening compound of the above-disclosed formula and from 0.1% to 2% by weight of a linear ethoxylated alcohol, and/or (from 0.1% to 2.5% by weight) other nonionic emulsifier, which help maintain stability during storage. The compositions also contain liquid carrier, e.g., water, preferably a mixture of a $C_1$-$C_4$ monohydric alcohol and water. Such liquid compositions are preferably formulated at a pH of from 2.0 to 5.0, preferably from 2.5 to 4, to provide good storage stability. For general laundry fabric softening use in a through-the-rinse mode, such compositions will typically comprise from 2% to 30% by weight of the fabric softening compound.

The preferred liquid compositions herein have the softening compound present as particles dispersed in the liquid carrier. The particles are preferably sub-micron size, generally having average diameters in the range of 0.10-0.50 microns. The materials that stabilize the compositions during storage, e.g., the linear alkoxylated alcohol also stabilize the dispersions against phase separation (i.e., creaming).

Importantly, the liquid compositions herein are substantially free (generally, less than 1%) of free (i.e., unprotonated) amines, since free amines can catalyze decomposition of the quaternized ester-amine softening compounds on storage. If minor amounts of amines are present, they should be protonated with acid during the formulation of the compositions. Relatively strong acids, such as $H_3PO_4$, citric, and HCl, can be used for this purpose.

The low viscosities exhibited by dispersions of particles of the softening compounds herein allow them to be formulated as water-dilutable fabric softener "high concentrates" which contain from 15% to 40% by weight of the fabric softener compound. Such high concentrates can be conveniently packaged for dilution with water by the user to produce "single-strength" softeners (typically, 3-10% concentration of softener active).

The invention also encompasses a method of softening fibers (including hair) or fabrics, or imparting an antistatic finish thereto, comprising contacting said fibers or fabrics with a compound of the above-disclosed type.

All percentages, ratios and proportions herein are by weight, unless otherwise specified.

## DETAILED DESCRIPTION OF THE INVENTION

The compounds of the present invention contain two ester linkages, with at least one ester linkage having the carbonyl carbon atom closer to the quaternary ammonium nitrogen atom (reverse ester). I.e., at least one hydrophobic group is provided by a fatty alcohol rather than a fatty acid.

### Quaternized Ester-Amine Softening Compound

The present invention contains as an essential component from 1% to 40%, preferably from 2% to 30%, of a quaternized ammonium softening compound having the formula:

$$\begin{array}{l} R_2-N^{\oplus}-[(CH_2)_n-C(O)-O-R^2]_m \quad X^{\ominus} \\ \quad\;\; | \\ \quad\; R^1{(2-m)} \end{array}$$

wherein each R substituent is a short chain ($C_1$-$C_6$, preferably $C_1$-$C_3$) alkyl or hydroxyalkyl group, e.g., methyl (most preferred), ethyl, propyl, hydroxyethyl, or mixtures thereof; $R^1$ is $-(CH_2)_n-O-(O)C-R^2$ or $R^2$; each $R^2$ is a long chain $C_{13}$-$C_{22}$ hydrocarbyl substituent, preferably $C_{13}$-$C_{17}$ alkyl; m is 1 or 2; and n is from 1 to 4. The counterion $X^{\ominus}$ is not critical herein, and can be any softener-compatible anion, for example, chloride, bromide, methyl- or ethylsulfate, formate, sulfate, nitrate and the like. It will be understood that substituents R, $R^1$ and $R^2$ can optionally be substituted with various groups such as alkoxyl, hydroxyl, can be saturated or unsaturated, or can be straight chain or branched. The compounds can be considered to be diester variations of ditallow dimethyl ammonium chloride (DTDMAC) which is a widely used fabric softener.

The above compounds used as the active softener and anti-static ingredient in the practice of this invention are prepared by the following processes. For example, in a typical synthesis, a fatty acid dimethylmonoethanol amine ester of the formula $R_2NCH_2CH_2O(O)CR^2$ is reacted with a chloroacetic acid ester of a fatty alcohol of the formula $R^2O(O)CCH_2Cl$, to yield the desired reaction product (wherein R and $R^2$ are as defined in the present application). Another typical synthesis involves reacting dimethyl amine sequentially with two moles of said chloroacetic acid ester of a fatty alcohol. However, it will be appreciated by those skilled in the chemical arts that this reaction sequence allows a broad selection of compounds to be prepared. As illustrative, nonlimiting, examples there can be mentioned the following quaternary ammonium diester compounds (wherein all long-chain alkyl substituents are straight-chain):

$[CH_3]_2N^{\oplus}[CH_2C(O)OC_{16}H_{33}]_2\ Br^{\ominus}$
$[CH_3]_2N^{\oplus}[CH_2C(O)OC_{17}H_{35}]_2\ Cl^{\ominus}$
$[C_2H_5]_2N^{\oplus}[CH_2CH_2O(O)CC_{13}H_{27}][CH_2C(O)OC_{17}H_{35}]\ Cl^{\ominus}$
$[C_2H_5][CH_3][C_{15}H_{31}C(O)OC_2H_4]^{\oplus}NCH_2CH_2OC(O)C_{14}H_{29}\ CH_3SO_4^{\ominus}$

In another synthesis of a diester variation of DTDMAC, an amine polycarboxylate of the formula $RN[CH_2C(O)OH]_2$ is esterified at both acid groups with a fatty alcohol of the formula $R^1OH$ then quaternized with an alkyl halide, RX, to yield the desired reaction product (wherein R and $R^1$ are as defined in the present application). A method for the synthesis of a preferred diester softening compound is disclosed in detail hereinafter. However, it will be appreciated by those skilled in the chemical arts that this reaction sequence allows a broad selection of compounds to be prepared. As illustrative, nonlimiting examples there can be mentioned the following (wherein all long-chain alkyl substituents are straight-chain):

$[HO-CH(CH_3)CH_2][CH_3]^{\oplus}N[CH_2C(O)OC_{16}H_{33}]_2\ Br^{\ominus}$
$[C_2H_5]2^{\oplus}\ N[CH_2C(O)OC_{18}H_{37}]_2\ Cl^{\ominus}$
$[CH_3][C_2H_5]^{\oplus}N[CH_2C(O)OC_{14}H_{29}]_2\ I^{\ominus}$
$[C_3H_7][C_2H_5]^{\oplus}N[CH_2C(O)OC_{16}H_{33}]_2\ CH_3SO_4^{\ominus}$
$[CH_3]_2^{\oplus}N-[CH_2C(O)OC_{16}H_{33}][CH_2C(O)OC_{18}H_{37}]\ Cl^{\ominus}$

Since the foregoing compounds are somewhat labile to hydrolysis, they should be handled rather carefully when used to formulate the compositions herein. For example, stable liquid compositions herein are formulated at a pH in the range of about 2.0 to 5.0, preferably about pH 3 ±0.5. The pH can be adjusted by the addition of a Bronsted acid. Examples of suitable Bronsted acids include the inorganic mineral acids, carboxylic acids, in particular the low molecular weight ($C_1$-$C_5$) carboxylic acids, and alkylsulfonic acids. Suitable inorganic acids include HCl, $H_2SO_4$, $HNO_3$ and $H_3PO_4$. Suitable organic acids include formic, citric, acetic, methylsulfonic and ethylsulfonic acid. Preferred acids are citric, hydrochloric, and phosphoric acids.

Synthesis of a Quaternized Ester Amine Softening Compound

Synthesis of one of the biodegradable, quaternized diester amine softening compounds herein is accomplished by the following two-step process:

Step A. Synthesis of (Stearyl) N,N-Dimethyl Aminoacetate

$$(CH_3)_2\text{-N-H} + ClCH_2C(O)OC_{18}H_{37} \rightarrow (CH_3)_2\text{-N-}CH_2C(O)OC_{18}H_{37}$$

The reaction was carried out in a three-necked-round-bottom flask equipped with temperature probe, mechanical agitator, and reflux condenser. The chloroacetate (343 g, 1.0 mole) is loaded; cooled down to 10°C, and then dimethylamine (DMA) is bubbled through the acetate. The reaction temperature is observed to increase rapidly to 57°C and the reaction mix turns slightly turbid. After 70 minutes of bubbling DMA (161 g, 3.6 moles) through the acetate the temperature reaches 100°C without any heat being applied. No more DMA is added after this and 30 minutes later a first sample (reaction temperature 48°C) is caught and analyzed with the following results: TAV (Total Amine Value, or Amine Number): 101, AV (Acid Value): 122, 3' amine value: nil. One hour after the first sample is taken the reaction mixture's temperature drops to 27°C. The bubbling DMA is restarted and after 15 minutes 161 (3.6 moles) more grams of DMA is bubbled through and the reaction temperature is only increased to 45°C. Heat is then applied to liquefy the pasty concoction. At 70°C this liquefaction is attained and the mixture is allowed to react for 3 more hours and 45 minutes at this same temperature before shutting it down and unloading the product for work-up. Analysis for the finished product is: TAV: 111, AV: 124, 3' amine value: nil.

In order to obtain the desired amine, in acceptable purity, the obtained product (amine hydrochloride) is neutralized and the DMA stripped off.

The amine hydrochloride is neutralized with a saturated aqueous solution of sodium carbonate. The required amount of sodium carbonate solution (proportionate to a 1:1 molar ratio of carbonate to amine salt) plus 200 ml of water are added to the molten amine salt and this mixture stirred for several minutes at room temperature. This mixture is then transferred into a separatory funnel where the water layer is allowed to separate, then drained and finally discarded.

The organic layer from the separatory funnel is analyzed with the following results: TAV: 143, AV: nil, moisture: 0.94%. Per TAV, product is presumed to be around 90% pure. The impurities are speculated to be either desired quaternary amine product or unreacted stearyl chloroacetate. In order to determine the identity of the impurity a sample of product is allowed to react at 90°C for 1 hour and then sampled to check TAV. The TAV dropped from 143 to 125 which leads one to believe that the impurity is mainly stearyl chloroacetate.

The reaction equation summarizing this second stage in the process of preparation of the desired quat is:

$$ROC(O)CH_2Cl + ROC(O)CH_2N(CH_3)_2 \rightarrow [ROC(O)CH_2]_2N^{\oplus}(CH_3)_2\ Cl^{\ominus}$$

The isolated N,N-dimethyl amino stearyl acetate from I. which is characterized as 90% pure is reacted with stearyl chloroacetate in a 1:1 molar ratio (corrected for the estimated 10% impurities) as detailed below:

Two hundred and four grams (0.47 moles) N,N-dimethyl amino stearyl acetate is combined with 102 grams (0.47 moles) of stearyl chloroacetate and 122 grams of denatured ethanol in a three-necked-round-bottom flask equipped with mechanical agitator, temperature probe, and reflux condenser. The mixture is then allowed to react at 83°C for 14 hours. Finished product is unloaded and analyzed to be as follows:

- Activity: 73.2% in ethanol
- Free Amine: 2.7%
- Gardner Color: 1+
- Ash: 0.24%

Synthesis of Another Quaternized Di-Ester Amine Softening Compound

Another biodegradable, quaternized diester amine fabric softening compound used in the present invention can be synthesized using the following two-step process:

Step A. Synthesis of Amine

$$(CH_3)_2\text{-}N\text{-}CH_2CH_2OH + ClC(O)C_{15}H_{31} \xrightarrow{(C_2H_5)_3N} (CH_3)_2\text{-}N\text{-}CH_2CH_2OC(O)C_{15}H_{31}$$

0.6 mole of dimethyl ethanol amine is placed in a 3-liter, 3-necked flask equipped with a reflux condenser, argon (or nitrogen) inlet and two addition funnels. In one addition funnel is placed 0.8 moles of triethylamine and in the second addition funnel is placed 0.6 moles of palmitoyl chloride in a 1:1 solution with methylene chloride. Methylene chloride (750 ml) is added to the reaction flask containing the amine and heated to 35°C (water bath). The triethylamine is added dropwise, and the temperature is raised to 40-45°C while stirring over one-half hour. The palmitoyl chloride/methylene chloride solution is added dropwise and allowed to heat at 40-45°C under inert atmosphere overnight (12-16 hrs.).

The reaction mixture is cooled to room temperature and diluted with chloroform (1500 ml). The chloroform solution of product is placed in a separatory funnel (4 L) and washed with sat. NaCl, dil. $Ca(OH)_2$, 50% $K_2CO_3$ (3 times)*, and, finally, sat. NaCl. The organic layer is collected and dried over $MgSO_4$ and filtered. Solvents are removed via rotary evaporation. Final drying is done under high vacuum (0.25 mm Hg).

Step B: Quaternization

$$(CH_3)_2\text{-}N\text{-}CH_2CH_2OC(O)C_{15}H_{31} + ClCH_2CO_2C_{17}H_{35}$$

$$\longrightarrow (CH_3)_2\text{-}N^{\oplus}\text{-}(CH_2CH_2OC(O)C_{15}H_{31})(CH_2CO_2C_{17}H_{35})\ Cl^{\ominus}$$

0.5 moles of the dimethyl ethanol palmitate amine from Step A is reacted as in the previous reaction to provide a quaternary ammonium salt.

Linear Ethoxylated Alcohol and Other Nonionic Surfactants

The present invention contains from 0.1% to 2%, preferably from 0.5% to 1%, of a linear ethoxylated alcohol or from 0.1% to 2.5% by weight of a nonionic emulsifier, or mixtures thereof. The linear ethoxylated alcohol improves the stability of the fabric softening composition by stabilizing the particulate dispersions of the softening compounds against phase separation.

Linear ethoxylated alcohols useful in the present invention are selected from the group consisting of the condensation products of $C_8$-$C_{20}$, preferably $C_{10}$-$C_{18}$, linear fatty alcohols with from 3 to 100 moles, preferably from 8 to 50, of ethylene oxide.

Examples of linear ethoxylated fatty alcohols of this type include Neodol 23-7 (the condensation product of $C_{12}$-$C_{13}$ linear alcohol with 7 moles ethylene oxide), Neodol 91-2.5 (the condensation product of $C_9$-$C_{11}$ linear alcohol with 2.5 moles ethylene oxide), Neodol 45-9 (the condensation product of $C_{14}$-$C_{15}$ linear alcohol with 9 moles of ethylene oxide), Neodol 45-7 (the condensation product of $C_{14}$-$C_{15}$ linear alcohol with 7 moles of ethylene oxide), Neodol 45-4 (the condensation product of $C_{14}$-$C_{15}$ linear alcohol with 4 moles of ethylene oxide), all of which are marketed by Shell Chemical Company, Genapol T-110 (the condensation product of tallow fatty alcohol with 11 moles of ethylene oxide), Brij-76 (the condensation product of tallow fatty alcohol and 10 moles of ethylene oxide), and Kyro EOB (the condensation product of $C_{13}$-$C_{15}$ linear alcohol with 9 moles ethylene oxide), marketed by The Procter & Gamble Company. Preferred are the condensation products of $C_{16}$-$C_{18}$ linear alcohols with from 8 to 50 moles of ethylene oxide, especially for concentrated compositions.

Performance and, usually, stability of the softener compositions decreases when the ethoxylated alcohol contains less than 8 ethoxy groups. Therefore, the preferred ethoxylated surfactants have an HLB (hydrophilic-lipophilic balance) of from 7 to 20, preferably from 8 to 15. Of course, by defining the length of the hydrophobic chain and the number of ethoxylate groups, the HLB of the surfactant is, in general, determined. However, it is to be noted that the nonionic

*Note: 50% $K_2CO_3$ layer will be below chloroform layer.

ethoxylated surfactants useful herein, for concentrated liquid compositions, contain relatively long chain $R^2$ groups and are relatively highly ethoxylated. While shorter alkyl chain surfactants having short ethoxylated groups may possess the requisite HLB, they are not as effective herein.

The deca-, undeca-, dodeca-, tetradeca-, and pentadeca- ethoxylates of n-hexadecanol, and n-octadecanol having an HLB within the range recited herein are useful viscosity/dispersibility modifiers in the context of this invention. Exemplary ethoxylated primary alcohols useful herein as the viscosity/dispersibility modifiers of the compositions are n-$C_{18}$EO(10); and n-$C_{10}$EO(11). The ethoxylates of mixed natural or synthetic alcohols in the "tallow" chain length range are also useful herein. Specific examples of such materials include tallow-alcohol-EO(11), tallowalcohol-EO(18), and tallowalcohol -EO(25). The use of nonionic emulsifiers can stabilize the compositions against phase separation. Such nonionics and their usage levels, have been disclosed in U.S. Pat. No. 4,454,049, MacGilp et al., issued June 12, 1984.

Specific examples of nonionic emulsifiers suitable for use in the compositions herein include fatty acid esters of glycerol (preferably glycerol monostearate) and fatty alcohols (e.g., stearyl alcohol). The standard nonionic emulsifiers, if used, are used at levels of from 0.1% to 2.5% by weight of the composition. Mixtures of glycerol monostearate with a linear ethoxylated alcohol are particularly preferred.

Liquid Carrier

The compositions herein comprise a liquid carrier, e.g., water. The compositions can also contain a small amount of a $C_1$-$C_4$ monohydric alcohol (e.g., ethanol, propanol, isopropanol, butanol, and mixtures thereof), ethanol being preferred. These compositions comprise from 60% to 99%, preferably from 70% to 95% of the liquid carrier. Preferably, the amount of the $C_1$-$C_4$ monohydric alcohol in the liquid carrier is up to 20%, preferably less than 10%, by weight of the quaternized ester-amine softening compound, the balance of the liquid carrier being water.

The softening compounds used in this invention are insoluble in such water-based carriers and, thus, are present as a dispersion of fine particles therein. These particles are sub-micron in size and are conveniently prepared by high-shear mixing which disperses the compounds as fine particles. A method of preparation of a preferred dispersion is disclosed in detail in Examples I-IV hereinafter. Again, since the softening compounds are hydrolytically labile, care should be taken to avoid the presence of base, and to keep the processing temperatures and pH within the ranges specified hereinafter.

Fully-formulated fabric softening compositions can contain, in addition to the rapidly biodegradable quaternary ester-amine compounds of the formula herein, linear alkoxylated fatty alcohol and liquid carrier, one or more of the following additional optional ingredients.

Conventional Quaternary Ammonium Softening Agents

The compositions of the present invention can further comprise a conventional di(higher alkyl) quaternary ammonium softening agent. The compositions herein can contain up to 25% (preferably from 0.1% to 10%) of the conventional di(higher alkyl)quaternary ammonium softening agent.

By "higher alkyl", as used in the context of the quaternary ammonium salts herein, is meant alkyl groups having from 8 to 30 carbon atoms, preferably from 11 to 22 carbon atoms. Examples of such conventional quaternary ammonium salts include:

(i) acyclic quaternary ammonium salts having the formula:

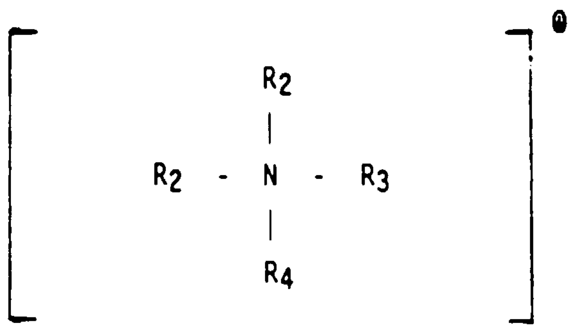

wherein $R_2$ is an acyclic aliphatic $C_{15}$-$C_{22}$ hydrocarbon group, $R_3$ is a $C_1$-$C_4$ saturated alkyl or hydroxyalkyl group, $R_4$ is selected from $R_2$ and $R_3$, and A is an anion;

(ii) diamido quaternary ammonium salts having the formula:

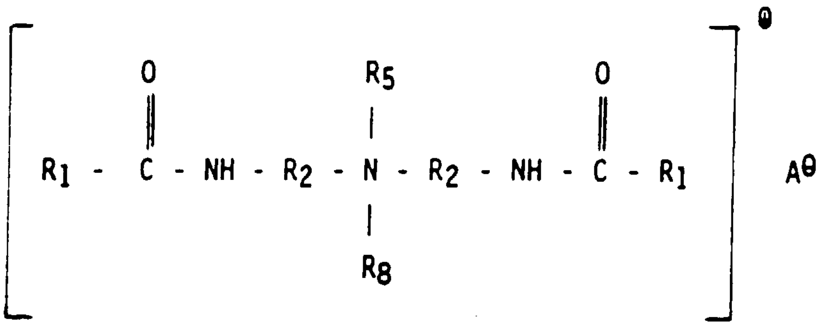

wherein $R_1$ is an acyclic aliphatic $C_{15}$-$C_{22}$ hydrocarbon group, $R_2$ is a divalent alkylene group having 1 to 3 carbon atoms, $R_5$ and $R_8$ are $C_1$-$C_4$ saturated alkyl or hydroxyalkyl groups, and A is an anion;

(iii) diamido alkoxylated quaternary ammonium salts having the formula:

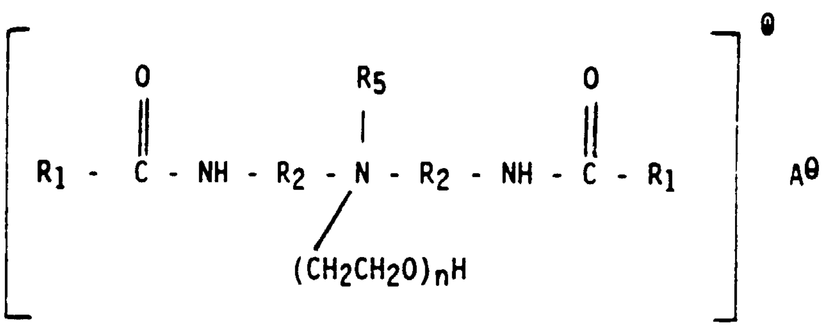

wherein n is equal to from about 1 to about 5, and $R_1$, $R_2$, $R_5$ and A are as defined above for compound (ii);

(iv) quaternary imidazolinium compounds having the formula:

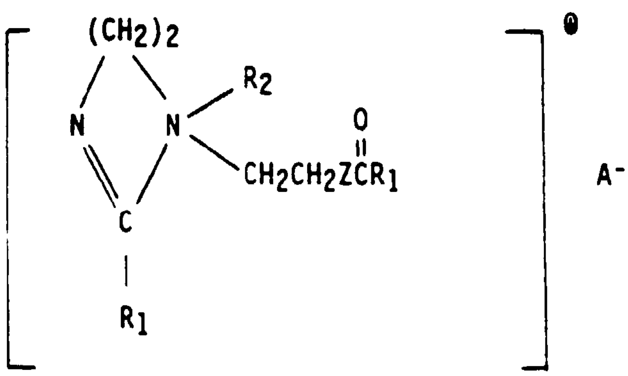

wherein $R_1$ = $C_{15}$-$C_{17}$ saturated alkyl, $R_2$ = $C_1$-$C_4$ saturated alkyl or H, Z = NH or 0, and A is an anion.

Examples of Component (i) are the well-known dialkyldimethylammonium salts such as ditallowdimethylammonium chloride, ditallowdimethylammonium methylsulfate, di(hydrogenated tallow) dimethylammonium chloride, dibehenyldimethylammonium chloride.

Examples of Components (ii) and (iii) are methylbis(tallow-amidoethyl) (2-hydroxyethyl) ammonium methylsulfate and methylbis-(hydrogenated tallowamidoethyl) (2-hydroxyethyl) ammonium methylsulfate, wherein $R_1$ is an acyclic aliphatic $C_{15}$-$C_{17}$ hydrocarbon group, $R_2$ is an ethylene group, $R_5$ is a methyl group, $R_8$ is a hydroxyalkyl group and A is a methylsulfate anion; these materials are available from Sherex Chemical Company under the trade names Varisoft® 222 and Varisoft® 110, respectively.

Examples of Component (iv) are 1-methyl-1-tallowamino-ethyl-2-tallowimidazolinium methylsulfate and 1-methyl-1-(hydrogenated tallowamidoethyl)-methylsulfate.

Free Amines

The liquid compositions herein should be substantially free (generally less than 1%) of free (i.e., unprotonated) amines. Care should be taken that if minor amounts of these amines are used to enhance the dispersion stability of the compositions, they are protonated with acid during formulation, otherwise the free amines may catalyze decomposition of the biodegradable quaternary ammonium compounds during storage.

Minor amounts of protonated amines, typically from 0.05% to 1.0%, namely primary, secondary and tertiary amines having, at least, one straight-chain organic group of from 12 to 22 carbon atoms may be used in the compositions of the present invention to enhance dispersion stability. Preferred amines of this class are ethoxyamines, such as monotallow-dipolyethoxyamine, having a total of from 2 to 30 ethoxy groups per molecule. Also suitable are diamines such as tallow-N,N', N'-tris (2-hydroxyethyl)-1,3-propylenediamine, or $C_{16}$-$C_{18}$-alkyl-N-bis(2-hydroxyethyl)amines.

Examples of the above compounds are those marketed under the trade names GENAMIN C, S, O and T, by Hoechst.

Di-(Higher Alkyl) Cyclic Amine

The compositions herein optionally comprise up to 25% (preferably from 0.1% to 10%) by weight of the composition of a di(higher alkyl) cyclic amine fabric softening agent of the formula:

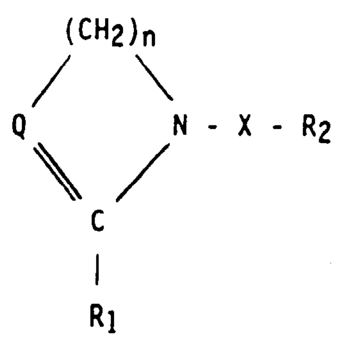

wherein n is 2 or 3, preferably 2; $R_1$ and $R_2$ are, independently, a $C_8$-$C_{30}$ alkyl or alkenyl, preferably $C_{11}$-$C_{22}$ alkyl, more preferably $C_{15}$-$C_{18}$ alkyl, or mixtures of such alkyl radicals. Examples of such mixtures are the alkyl radicals obtained from coconut oil, "soft" (non-hardened) tallow, and hardened tallow. Q is CH or N, preferably N. X is $R_4$-T-C-(O)- wherein T is O or $NR_5$, $R_5$ being H or $C_1$-$C_4$ alkyl, preferably H, and $R_4$ is a divalent $C_1$-$C_3$ alkylene group or $(C_2H_4O)_m$, wherein m is from 1 to 8.

Silicone Component

The fabric softening compositions herein optionally contain an aqueous emulsion of a predominantly linear polydialkyl or alkyl aryl siloxane in which the alkyl groups can have from one to five carbon atoms and may be wholly or partially fluorinated. These siloxanes act to provide improved absorbency benefits. Suitable silicones are polydimethyl siloxanes having a viscosity, at 25°C, of from 1 to 100,000 centistokes, preferably from 1,000 to 12,000 centistokes. For some applications, low (~1 cst) viscosity silicones are preferred.

The fabric softening compositions herein can contain up to 15%, preferably from 0.1% to 10%, of the silicone component.

Thickening Agent

Optionally, the compositions herein contain up to 3%, preferably from 0.01% to 2%, of a thickening agent. Examples of suitable thickening agents include: cellulose derivatives, synthetic high molecular weight polymers (e.g., carboxyvinyl polymer and polyvinyl alcohol), and cationic guar gums.

The cellulosic derivatives that are functional as thickening agents herein may be characterized as certain hydroxyethers of cellulose, such as Methocel[K], marketed by Dow Chemicals, Inc.; also, certain cationic cellulose ether derivatives, such as Polymer JR-125®, JR-400®, and JR-30M®, marketed by Union Carbide.

Other effective thickening agents are cationic guar gums, such as Jaguar Plus®, marketed by Stein Hall, and Gendrive 458®, marketed by General Mills.

Preferred thickening agents herein are selected from the group consisting of methyl cellulose, hydroxypropyl methylcellulose, or hydroxybutyl methylcellulose, said cellulosic polymer having a viscosity in 2% aqueous solution at 20°C of from 15 to 75,000 centipoise.

Soil Release Agent

Optionally, the compositions herein contain from 0% to 10%, preferably from 0.2% to 5%, of a soil release agent. Preferably, such a soil release agent is a polymer. Polymeric soil release agents useful in the present invention include copolymeric blocks of terephthalate and polyethylene oxide or polypropylene oxide.

A preferred soil release agent is a copolymer having blocks of terephthalate and polyethylene oxide. More specifically, these polymers are comprised of repeating units of ethylene terephthalate and polyethylene oxide terephthalate at a molar ratio of ethylene terephthalate units to polyethylene oxide terephthalate units of from 25:75 to 35:65, said polyethylene oxide terephthalate containing polyethylene oxide blocks having molecular weights of from 300 to 2000. The molecular weight of this polymeric soil release agent is in the range of from 5,000 to 55,000.

Another preferred polymeric soil release agent is a crystallizable polyester with repeat units of ethylene terephthalate units containing from 10% to 15% by weight of ethylene terephthalate units together with from 10% to 50% by weight of polyoxyethylene terephthalate units, derived from a polyoxyethylene glycol of average molecular weight of from 300 to 6,000, and the molar ratio of ethylene terephthalate units to polyoxyethylene terephthalate units in the crystallizable polymeric compound is between 2:1 and 6:1. Examples of this polymer include the commercially available materials Zelcon® 4780 (from DuPont) and Milease® T (from ICI).

Highly preferred soil release agents are polymers of the generic formula:

$$X-(OCH_2CH_2)_n(O-\overset{\overset{O}{\|}}{C}-R^1-\overset{\overset{O}{\|}}{C}-OR^2)_u(O-\overset{\overset{O}{\|}}{C}-R^1-\overset{\overset{O}{\|}}{C}-O)\ (CH_2CH_2O-)_n-X$$

in which X can be any suitable capping group, with each X being selected from the group consisting of H, and alkyl or acyl groups containing from 1 to 4 carbon atoms. n is selected for water solubility and generally is from 6 to 113, preferably from 20 to 50. u is critical to formulation in a liquid composition having a relatively high ionic strength. There should be very little material in which u is greater than 10. Furthermore, there should be at least 20%, preferably at least 40%, of material in which u ranges from 3 to 5.

The $R^1$ moieties are essentially 1,4-phenylene moieties. As used herein, the term "the $R^1$ moieties are essentially 1,4-phenylene moieties" refers to compounds where the $R^1$ moieties consist entirely of 1,4-phenylene moieties, or are partially substituted with other arylene or alkarylene moieties, alkylene moieties, alkenylene moieties, or mixtures thereof. Arylene and alkarylene moieties which can be partially substituted for 1,4phenylene include 1,3-phenylene, 1,2-phenylene, 1,8-naphthylene, 1,4-naphthylene, 2,2-biphenylene, 4,4-biphenylene and mixtures thereof. Alkylene and alkenylene moieties which can be partially substituted include ethylene, 1,2-propylene, 1,4-butylene, 1,5-pentylene, 1,6-hexamethylene, 1,7-heptamethylene, 1,8-octamethylene, 1,4-cyclohexylene, and mixtures thereof.

For the $R^1$ moieties, the degree of partial substitution with moieties other than 1,4-phenylene should be such that the soil release properties of the compound are not adversely affected to any great extent. Generally, the degree of partial substitution which can be tolerated will depend upon the backbone length of the compound, i.e., longer backbones can have greater partial substitution for 1,4-phenylene moieties. Usually, compounds where the $R^1$ comprise from 50% to 100% 1,4-phenylene moieties (from 0 to 50% moieties other than 1,4-phenylene) have adequate soil release activity. For example, polyesters made according to the present invention with a 40:60 mole ratio of isophthalic (1,3-phenylene) to terephthalic (1,4-phenylene) acid have adequate soil release activity. However, because most polyesters used in fiber making comprise ethylene terephthalate units, it is usually desirable to minimize the degree of partial substitution with moieties other than 1,4-phenylene for best soil release activity. Preferably, the $R^1$ moieties consist entirely of (i.e., comprise 100%) 1,4-phenylene moieties, i.e., each $R^1$ moiety is 1,4-phenylene.

For the $R^2$ moieties, suitable ethylene or substituted ethylene moieties include ethylene, 1,2-propylene, 1,2-butylene, 1,2-hexylene, 3-methoxy-1,2-propylene and mixtures thereof. Preferably, the $R^2$ moieties are essentially ethylene moieties, 1,2-propylene moieties or mixture thereof. Inclusion of a greater percentage of ethylene moieties tends to improve the soil release activity of compounds. Surprisingly, inclusion of a greater percentage of 1,2-propylene moieties tends to improve the water solubility of the compounds.

Therefore, the use of 1,2-propylene moieties or a similar branched equivalent is desirable for incorporation of any substantial part of the soil release component in the liquid fabric softener compositions. Preferably, from 75% to 100%,

more preferably from 90% to 100%, of the $R^2$ moieties are 1,2-propylene moieties.

The value for each n is at least 6, and preferably is at least 10. The value for each n usually ranges from 12 to 113. Typically, the value for each n is in the range of from 12 to 43.

A more complete disclosure of these highly preferred soil release agents is contained in European Patent Application 185,427, Gosselink, published June 25, 1986.

Viscosity Control Agents

Viscosity control agents can be used in the compositions of the present invention (preferably in concentrated compositions). Examples of organic viscosity modifiers are fatty acids and esters, fatty alcohols, and water-miscible solvents such as short chain alcohols. Examples of inorganic viscosity control agents are water-soluble ionizable salts. A wide variety of ionizable salts can be used. Examples of suitable salts are the halides of the group IA and IIA metals of the Periodic Table of the Elements, e.g., calcium chloride, magnesium chloride, sodium chloride, potassium bromide, and lithium chloride. Calcium chloride is preferred. The ionizable salts are particularly useful during the process of mixing the ingredients to make the compositions herein, and later to obtain the desired viscosity. The amount of ionizable salts used depends on the amount of active ingredients used in the compositions and can be adjusted according to the desires of the formulator. Typical levels of salts used to control the composition viscosity are from 20 to 5,000 parts per million (ppm), preferably from 1,000 to 3,000 ppm, by weight of the composition.

Bactericides

Examples of bactericides used in the compositions of this invention include glutaraldehyde, formaldehyde, 2-bromo-2-nitropropane-1,3-diol sold by Inolex Chemicals under the trade name Bronopol®, and a mixture of 5-chloro-2-methyl-4-isothiazoline-3-one and 2-methyl-4-isothiazoline-3-one sold by Rohm and Haas Company under the trade name Kathon® CG/ICP. Typical levels of bacteriocides used in the present compositions are from 1 to 1,000 ppm by weight of the composition.

Other Optional Ingredients

The present invention can include other optional components conventionally used in textile treatment compositions, for example, colorants, perfumes, preservatives, optical brighteners, opacifiers, fabric conditioning agents, surfactants, stabilizers such as guar gum and polyethylene glycol, anti-shrinkage agents, anti-wrinkle agents, fabric crisping agents, spotting agents, germicides, fungicides, anti-oxidants such as butylated hydroxy toluene, anti-corrosion agents.

In the method aspect of this invention, fabrics or fibers are contacted with an effective amount, generally from 20 ml to 200 ml (per 3.5 kg of fiber or fabric being treated), of the compositions herein in an aqueous bath. Of course, the amount used is based upon the judgment of the user, depending on concentration of the composition, fiber or fabric type, degree of softness desired. Typically, 90 mls of a 5% dispersion of the softening compounds are used in a 95 l (25 gallon) laundry rinse bath to soften and provide antistatic benefits to a 3.5 kg load of mixed fabrics. Preferably, the rinse bath contains from 25 ppm to 100 ppm of the fabric softening compositions herein.

The following examples illustrate the practice of the present invention but are not intended to be limiting thereof.

EXAMPLE I

A storage stable biodegradable fabric softening composition of the present invention is made as follows:

| Ingredient | Percent (wt.) |
|---|---|
| $(CH_3)_2\text{-}^{\oplus}N\text{-}[CH_2C(O)OC_{16}H_{33}]_2\ Cl^{\ominus}$ | 5.0 |
| Isopropanol | 1.0 |
| Glyceryl Monostearate (GMS) | 1.2 |
| Neodol 23-3 | 0.5 |
| Bronopol | 0.01 |
| Dye | 20 ppm |
| 0.1 N HCl | 0.25 |
| Water | Balance |

20 g of the biodegradable diester amine softener compound and 5 g of isopropanol are mixed and heated to 80°C

to form a fluidized "melt." 4.8 g of GMS and 2 g Neodol 23-3 are then added to the melt to form a homogeneous molten mixture. The molten mixture is then poured into a 400 g water seat with high shear mixing. The water is preheated to 70°C, and 20 ppm blue dye and 100 ppm Bronopol are added to the water prior to mixing. About 1 g of isopropanol is evaporated from the molten mixture before it is poured into the water. The dispersion is mixed for 25 minutes at 7000 rpm (Tekmar® high shear mixer). During mixing the temperature of the dispersion is maintained within 70-75°C by a cooling water bath. The pH is adjusted by the addition of 1 ml of 0.1 N HCl.

EXAMPLE II

A storage stable biodegradable fabric softening composition of the present invention is made as follows:

| Ingredient | Percent (wt.) |
|---|---|
| $(CH_3)_2-{}^{\oplus}N-[CH_2CH_2OC(O)C_{15}H_{31}][CH_2C(O)OC_{16}H_{33}]\ Cl^{\ominus}$ | 5 |
| Isopropanol | 1.1 |
| Glyceryl Monostearate (GMS) | 1 |
| Neodol 23-3 | 1 |
| 0.1 N HCl | 0.25 |
| Water | Balance |

20 g of the biodegradable diester amine softener compound and 5 g of isopropanol are mixed and heated to 75°C to form a fluidized "melt." Four g of GMS and 4 g of Neodol 23-3 are then added to the melt to form a homogeneous molten mixture. The molten mixture is then poured into a 365 g water seat with high shear mixing. The water is preheated to 70°C. 0.6 g of isopropanol is evaporated from the molten mixture before it is poured into the water. The dispersion is mixed for 20 minutes at 7200 rpm (Tekmar high shear mixer). The pH is adjusted by the addition of 1 ml of 0.1 N HCl.

EXAMPLE III

A storage stable biodegradable fabric softening composition of the present invention is made as follows:

| Ingredient | Percent (wt.) |
|---|---|
| $(CH_3)_2-N^{\oplus}-[CH_2C(O)OC_{18}H_{37}]_2\ Cl^{\ominus}$ | 4.5 |
| Isopropanol | 0.6 |
| Glyceryl Monostearate (GMS) | 1.2 |
| Neodol 23-3 | 0.3 |
| Polydimethylsiloxane (PDMS) | 0.1 |
| 0.1 N HCl | 0.25 |
| Water | Balance |

18 g of the biodegradable diester amine softener compound and 2.4 g of isopropanol are mixed and heated to 75°C to form a fluidized "melt." 4.8 g of GMS and 1.2 g of Neodol 23-3 are then added to the melt to form a homogeneous molten mixture. The molten mixture is then poured into a 375 g water seat with high shear mixing. The water is preheated to 70°C. The dispersion is mixed for 15 minutes at 7000 rpm (Tekmar high shear mixer). After the dispersion cools down to about 30°C, 0.4 g of PDMS is added to the dispersion with low shear mixing (3000 rpm for 3 minutes). The pH is adjusted by the addition of 1 ml of 0.1 N HCl.

EXAMPLE IV

A storage stable biodegradable concentrated fabric softening composition of the present invention is made as follows:

| Ingredient | Percent (wt.) |
|---|---|
| $(CH_3)_2-N^{\oplus}-[CH_2C(O)OC_{18}H_{37}]_2\ Cl^{\ominus}$ | 15 |
| Isopropanol | 2.5 |
| Glycerol Monostearate (GMS) | 1.0 |

(continued)

| Ingredient | Percent (wt.) |
|---|---|
| Neodol 23-3 | 0.5 |
| $CaCl_2$ | 0.06 |
| 0.1 N HCl | 0.25 |
| Water | Balance |

30 g of the biodegradable diester amine softener compound and 5 g of isopropanol are mixed and heated to 75°C to form a fluidized melt. Two g of GMS and 1 g of Neodol 23-3 are then added to the melt to form a homogeneous molten mixture. The melt is then poured into a 165 g water seat with high shear mixing. The water is preheated to 60°C. The dispersion is mixed for 15 minutes at 7000 rpm (Tekmar high shear mixer). 6 ml of 2% $CaCl_2$ aqueous solution is added to the dispersion during mixing to prevent the dispersion from gelling. During mixing the dispersion' temperature is maintained at about 60°C. The pH is adjusted by the addition of 0.5 ml of 0.1 N HCl.

In a convenient mode, this concentrated composition is packaged in a simple plastic pouch, which is opened and poured into 4X its volume of water prior to use to prepare a "single strength" softener composition, thereby saving on packaging and shipping costs, as well storage space.

Typically, the liquid fabric softening compositions in the above examples are added to the rinse cycle of conventional washing machines. When multiple rinses are used, the fabric softening composition is preferably added to the final rinse. The amount added to the rinse cycle is generally from about 20 ml to about 200 ml (per 3.5 kg of fabric being treated) of the compositions of Examples I-III (and the diluted version of Example IV).

In all of the above examples, substantially similar results are obtained when Neodol 23-3 is replaced, in whole or in part, with Neodol 45-9 (the condensation product of $C_{14}$-$C_{15}$ linear alcohol with 9 moles of ethylene oxide), Neodol 45-7 (the condensation product of $C_{14}$-$C_{15}$ linear alcohol with 7 moles of ethylene oxide), Neodol 91-2.5 (the condensation product of $C_9$-$C_{11}$ linear alcohol with 2.5 moles ethylene oxide), Neodol 45-4 (the condensation product of $C_{14}$-$C_{15}$ linear alcohol with 4 moles of ethylene oxide), Genapol T-110, Brij-76, and Kyro EOB (the condensation product of $C_{13}$-$C_{15}$ linear alcohol with 9 moles ethylene oxide).

Similar results are obtained in Examples I-IV when the biodegradable quaternary diester amine softening compound is replaced, in whole or in part, with any of the following biodegradable quaternary diester amine softening compounds:

$[CH_3]_2N^{\oplus}[CH_2C(O)OC_{16}H_{33}]_2\ Br^{\ominus}$
$[CH_3]_2N^{\oplus}[CH_2C(O)OC_{17}H_{35}]_2\ Cl^{\ominus}$
$[C_2H_5]_2N^{\oplus}[CH_2CH_2O(O)CC_{13}H_{27}][CH_2C(O)OC_{17}H_{35}]\ Cl^{\ominus}$
$[C_2H_5][CH_3][C_{15}H_{31}C(O)OC_2H_4]^{\oplus}NCH_2CH_2OC(O)C_{14}H_{29}\ CH_3SO_4^{\ominus}$
$[HO\text{-}CH(CH_3)CH_2][CH_3]^{\oplus}N[CH_2C(O)OC_{16}H_{33}]_2\ Br^{\ominus}$
$[C_2H_5]_2{}^{\oplus}N[CH_2C(O)OC_{18}H_{37}]_2\ Cl^{\ominus}$
$[CH_3][C_2H_5]^{\oplus}N[CH_2C(O)OC_{14}H_{29}]_2\ I^{\ominus}$
$[C_3H_7][C_2H_5]^{\oplus}N[CH_2C(O)OC_{16}H_{33}]_2\ CH_3SO_4^{\ominus}$
$[CH_3]_2{}^{\oplus}\ N\text{-}[CH_2C(O)OC_{16}H_{33}][CH_2C(O)OC_{18}H_{37}]\ Cl^{\ominus}$

Similar results are also obtained when isopropanol in the above examples is replaced, in whole or in part, with ethanol, propanol, butanol, or mixtures thereof and when HCl is replaced, in whole or in part, with $H_3PO_4$ and/or citric acids.

Importantly, the above biodegradable compositions display excellent softening characteristics on both natural and synthetic fabrics, low viscosity at both normal and elevated temperatures, and good product stability and dispersibility, compared with compositions containing no linear ethoxylated alcohol.

## Claims

**1.** A liquid fabric softening and antistatic composition formulated at a pH of from 2.0 to 5.0 and comprising:

(a) from 1 to 40% by weight of a quaternized ester amine softening compound having the formula:

$$(R)_2\text{-}N^+\text{-}[(R^1{}_{(2-m)}]\ [(CH_2)_n\text{-}C(O)\text{-}O\text{-}R^2]_m\ X^-$$

wherein each R substituent is a short chain $C_1$-$C_6$ alkyl or hydroxyalkyl group or mixtures thereof;

$R^1$ is $-(CH_2)n-O-(O)C-R^2$ or $R^2$ group;
$R^2$ is a $C_{13}$-$C_{22}$ hydrocarbyl groups;
R, $R^1$ and $R^2$ can optionally be substituted with various groups such as alkoxy, hydroxyl, can be saturated or unsaturated, or can be straight chain or branched;
m is 1 or 2;
n is from 1 to 4; and
X is a softener compatible anion;

(b) a stabilising agent selected from a linear ethoxylated alcohol in amount of from 0.1% to 2% by weight, a nonionic emulsifier in amount of from 0.1% to 2.5% by weight and mixtures thereof;
(c) from 60% to 99% by weight of a liquid carrier.

**2.** A liquid fabric softening and antistatic composition according to Claim 1, wherein m is 2.

**3.** A liquid fabric softening and antistatic composition according to Claim 1, wherein m is 1 and $R^1$ is $-(CH_2)n-O-(O)C-R^2$.

**4.** A liquid fabric softening and antistatic composition according to any one of Claim 1-3, wherein said softening compound is in amount of from 2% to 10% by weight.

**5.** A liquid fabric softening and antistatic composition according to any one of claim 1-4, wherein the liquid carrier comprises a mixture of a $C_1$-$C_4$ monohydric alcohol, together with water, the amount of the monohydric alcohol being up to 20% by weight of the softening compound, the balance of the liquid carrier being water.

**6.** A composition according to any one of claims 1-5 wherein the softening compound is present as particles dispersed in the carrier.

**7.** A composition according to any of Claims 1-6 wherein the particles have an average diameter in the range of from 0.1 to 0.5 microns.

**8.** A composition according to any of Claims 1-7 which is substantially free (less than 1%) of free amines.

**9.** A composition according to any of Claims 1-8 in concentrated form which contains from 11% to 25% of the softening compound.

**10.** A composition according to any of Claims 1-9 which additionally contains from 20 to 3,000 ppm of a salt selected from the group consisting of calcium chloride, magnesium chloride, sodium chloride, potassium bromide, lithium chloride, and mixtures thereof, preferably calcium chloride.

**11.** A method of softening or providing an antistatic finish to fibers or fabrics by contacting said fibers or fabrics with an effective amount of the composition of Claim 1.

**Patentansprüche**

**1.** Flüssige textilweichmachende und antistatische Zusammensetzung, die bei einem pH von 2,0 bis 5.0 formuliert ist und

(a) 1 - 40 Gew.-% einer quaternisierten Esteramin-Weichmacherverbindung der Formel

$$(R)_2-N^+-[(R^1{}_{(2-m)})][(CH_2)_n-C(O)-O-R^2]_m X^-$$

worin jeder R-Substituent eine kurzkettige $C_1$-$C_6$-Alkyl- oder Hydroxyalkylgruppe oder Mischungen hiervon ist;

$R^1$ eine $-(CH_2)_n-O-(O)\ C-R^2$- oder $R^2$-Gruppe ist;

$R^2$ eine $C_{13}$-$C_{22}$-Hydrocarbylgruppe ist; wobei R, $R^1$, $R^2$ wahlweise mit verschiedenen Gruppen, wie Alkoxy, Hydroxyl, substituiert, gesättigt oder ungesättigt oder geradkettig oder verzweigt sein können;
m 1 oder 2 ist;
n 1 bis 4 ist; und
X ein weichmacherverträgliches Anion ist;

(b) ein Stabilisierungsmittel, gewählt aus einem linearen ethoxylierten Alkohol in einer Menge von 0,1 bis 2 Gew. -%, einem nichtionischen Emulgiermittel in einer Menge von 0,1 bis 2,5 Gew.-% und Mischungen hiervon;
(c) 60 bis 99 Gew.-% eines flüssigen Trägers umfaßt.

**2.** Flüssige textilweichmachende und antistatische Zusammensetzung nach Anspruch 1, wobei m 2 ist.

**3.** Flüssige textilweichmachende und antistatische Zusammensetzung nach Anspruch 1, wobei m 1 ist und $R^1$ die Bedeutung $-(CH_2)_n$-O-(O)C-$R^2$ hat.

**4.** Flüssige textilweichmachende und antistatische Zusammensetzung nach mindestens einem der Ansprüche 1 - 3, wobei die Weichmacherverbindung in einer Menge von 2 bis 10 Gew.-% vorliegt.

**5.** Flüssige textilweichmachende und antistatische Zusammensetzung nach mindestens einem der Ansprüche 1 - 4, wobei der flüssige Träger eine Mischung aus einem einwertigen $C_1$-$C_4$- Alkohol, zusammen mit Wasser umfaßt, wobei die Menge des einwertigen Alkohols bis zu 20 Gew. -% der Weichmacherverbindung beträgt, und wobei der Rest des flüssigen Trägers Wasser ist.

**6.** Zusammensetzung nach mindestens einem der Ansprüche 1 5, wobei die Weichmacherverbindung in Form von in dem Träger dispergierten Teilchen vorliegt.

**7.** Zusammensetzung nach mindestens einem der Ansprüche 1 - 6, wobei die Teilchen einen Durchschnittsdurchmesser im Bereich von 0,1 - 0,5 µm aufweisen.

**8.** Zusammensetzung nach mindestens einem der Ansprüche 1 - 7, welche im wesentlichen frei (weniger als 1 %) an freien Aminen ist.

**9.** Zusammensetzung nach mindestens einem der Ansprüche 1 - 8 in konzentrierter Form, welche 11 bis 25 % der Weichmacherverbindung enthält.

**10.** Zusammensetzung nach mindestens einem der Ansprüche 1 - 9, welche weiterhin 20 - 3000 ppm eines Salzes enthält, gewählt aus der Calciumchlorid, Magnesiumchlorid, Natriumchlorid, Kaliumbromid, Lithiumchlorid und Mischungen hiervon umfassenden Gruppe, vorzugsweise Calciumchlorid.

**11.** Verfahren zum Weichmachen oder Vorsehen einer antistatischen Ausrüstung bei Fasern oder Textilien durch Kontaktieren der Fasern oder Textilien mit einer wirksamen Menge der Zusammensetzung gemäß Anspruch 1.

## Revendications

**1.** Composition adoucissante et antistatique liquide pour le linge, formulée à un pH de 2,0 à 5,0 et comprenant:

(a) de 1 à 40% en poids d'un composé adoucissant de type ester-amine quaternisée, de formule:

$$(R)_2\text{-}N^+\text{-}[(R^1{}_{(2-m)}][(CH_2)_n\text{-}C(O)\text{-}O\text{-}R^2)_m\ X^-$$

dans laquelle chaque substituant R est un groupe alkyle ou hydroxyalkyle à chaîne courte en $C_1$-$C_6$, ou un mélange de ceux-ci;

$R^1$ est un groupe $(CH_2)_n$-O-C(O)-$R^2$ ou $R^2$;
$R^2$ est un groupe hydrocarboné en $C_{13}$-$C_{22}$;
R, $R^1$ et $R^2$ peuvent être éventuellement substitués par différents groupes tels que alcoxy, hydroxyle,

peuvent être saturés ou insaturés, ou peuvent être à chaîne droite ou ramifiée;
m vaut de 1 à 2;
n vaut de 1 à 4; et
X est un anion compatible avec l'adoucissant;

(b) un agent stabilisant choisi parmi un alcool éthoxylé linéaire, à raison de 0,1% à 2% en poids, un émulsifiant non ionique, à raison de 0,1% à 2,5% en poids, et leurs mélanges;
(c) de 60% à 99% en poids d'un véhicule liquide.

**2.** Composition adoucissante et antistatique liquide pour le linge selon la revendication 1, dans laquelle m vaut 2.

**3.** Composition adoucissante et antistatique liquide pour le linge selon la revendication 1, dans laquelle m vaut 1 et $R^1$ est $-(CH_2)_n-O-C(O)-R^2$.

**4.** Composition adoucissante et antistatique liquide pour le linge selon l'une quelconque des revendications 1-3, dans laquelle ledit composé adoucissant est présent à raison de 2% à 10% en poids.

**5.** Composition adoucissante et antistatique liquide pour le linge selon l'une quelconque des revendications 1-4, dans laquelle le véhicule liquide comprend un mélange d'un monoalcool en $C_1$-$C_4$, avec de l'eau, la quantité du monoalcool pouvant aller jusqu'à 20% en poids du composé adoucissant, le restant du véhicule liquide étant de l'eau.

**6.** Composition selon l'une quelconque des revendications 1-5, dans laquelle le composé adoucissant est présent sous forme de particules dispersées dans le véhicule.

**7.** Composition selon l'une quelconque des revendications 1-6, dans laquelle les particules ont un diamètre moyen dans la gamme de 0,1 à 0,5 micron.

**8.** Composition selon l'une quelconque des revendications 1-7, essentiellement dépourvue d'amines libres (moins de 1%).

**9.** Composition selon l'une quelconque des revendications 1-8, sous une forme concentrée qui contient de 11% à 25% du composé adoucissant.

**10.** Composition selon l'une quelconque des revendications 1-9, qui contient, en outre, de 20 à 3 000 ppm d'un sel choisi dans le groupe constitué par le chlorure de calcium, le chlorure de magnésium, le chlorure de sodium, le bromure de potassium, le chlorure de lithium, et leurs mélanges, de préférence le chlorure de calcium.

**11.** Procédé pour adoucir ou conférer un fini antistatique à des fibres ou des tissus, consistant à mettre en contact lesdites fibres ou lesdits tissus avec une quantité efficace de la composition de la revendication 1.